# EUROPÄISCHE PATENTSCHRIFT

(11) **EP 1 420 835 B1**
(45) Veröffentlichungstag und Bekanntmachung des Hinweises auf die Patenterteilung: **25.04.2007**
(21) Anmeldenummer: 02752933.8
(22) Anmeldetag: 23.08.2002
(51) Int. Cl.: A61M 1/00

(54) **IMPLANTAT MIT OBERFLÄCHENSTRUKTUR**
IMPLANT WITH A SURFACE STRUCTURE
IMPLANT A STRUCTURE SUPERFICIELLE

(30) Priorität: 31.08.2001 DE 10142637
(43) Veröffentlichungstag der Anmeldung: 26.05.2004
(73) Patentinhaber: Disetronic Licensing AG, 3401 Burgdorf (CH)
(72) Erfinder: REINMANN, Andreas, 3014 Bern (CH)
(74) Vertreter: Gassenhuber, Andreas
(86) Internationale Anmeldenummer: PCT/CH2002/000461
(87) Internationale Veröffentlichungsnummer: WO 2003/020342

(56) Entgegenhaltungen:
- US-A- 3 638 649
- US-A- 5 098 398
- US-A- 5 370 698
- US-A- 5 507 815
- US-B1- 6 270 475

## Beschreibung

Die vorliegende Erfindung bezieht sich auf ein Implantat mit einer Oberflächenstruktur, insbesondere auf ein transkutanes Implantat mit einer gerichteten Struktur auf der Oberfläche, welche das Einwachsverhalten des Implantats verbessert sowie ein Verfahren zur Herstellung eines solchen Implantats.

Es sind Implantate bekannt, welche in einen Körper vollständig oder zum Teil eingesetzt werden, wobei zum Beispiel bei einem eingesetzten Portkörper ein Bereich des Portkörpers aus der Hautoberfläche vorsteht. Hierbei ergeben sich häufig Probleme beim Anwachsen der umgebenden Haut an den Portkörper. Häufig bilden sich im Grenzbereich zwischen umgebender Haut und Portkörper Entzündungen, die sich entlang des eingesetzten Portkörpers in den Körper eines Patienten hinein ausbreiten. Dies ist für einen Patienten unangenehm und führt zu einem schlechten Sitz des Portkörpers und zu einem hohen Infektionsrisiko für den Patienten.

Aus der US 5,833,641 ist eine Vorrichtung zur Förderung einer Wundheilung bekannt, welche aus einem biologisch verträglichem Material besteht und biologisch abbaubar ist. Diese Vorrichtung wird zur Wundheilung bei der Wunde eingesetzt und weist eine Oberflächenstruktur auf, welche zur Orientierung eines gewünschten Zellwachstums dient und kann während oder nach der Verwendung biologisch abgebaut, also vom Patienten resorbiert werden.

Aus der US 5,098,398, die den nächstliegenden Stand der Technik repräsentiert, ist ein implantierbarer Durchgang bekannt, wobei, wenn das Element bei einem Menschen oder einem Tier implantiert wird, ein Abschnitt eines Rohrstückes aus der Epithelschicht herausragt, während vorstehende Flanschränder, eine Grundplatte und ein Gitter unter der Epithelschicht liegen. Um ein zufriedenstellendes Wachstum des verbindenden Gewebes in die vorstehenden Flanschränder zu erreichen, soll der Abstand zwischen den vorstehenden Flanschrändern mindestens 0,08 mm betragen und die Tiefe der Rille zwischen den nebeneinanderliegenden Flanschrändern soll mindestens 0,08 mm betragen.

Es ist eine Aufgabe der vorliegenden Erfindung ein Implantat vorzuschlagen, welches nach dem Einsetzen für einen Patienten verträglicher ist. Weiterhin soll ein Verfahren zur Herstellung des Implantats vorgeschlagen werden.

Diese Aufgabe wird durch das Implantat und das Verfahren zur Herstellung des Implantats mit den Merkmalen der unabhängigen Ansprüche gelöst. Vorteilhafte Ausführungsformen ergeben sich aus den Unteransprüchen.
Das erfindungsgemäße Implantat ist bevorzugt ein transkutanes Implantat, d. h. ein Implantat, welches nicht vollständig in einen Körper eingesetzt wird, so dass nach dem Einsetzen des Implantats mindestens ein Teil aus der Hautoberfläche vorsteht, wie zum Beispiel bei einem Portkörper, welcher zum Einbringen einer Substanz in ein unter der Hautoberfläche liegendes Gewebe dient. Erfindungsgemäß ist auf mindestens einem Oberflächenbereich des Implantats eine Oberflächenstruktur vorgesehen, welche das Einwachsverhalten des Implantats verbessert. Dabei ist die Oberflächenstruktur vorzugsweise so gerichtet, dass das Wachstum von um das Implantat liegendem Zellgewebe gefördert und/oder in einer oder mehreren bestimmten Vorzugsrichtungen unterstützt wird. Bei dem umliegenden Gewebe kann es sich zum Beispiel um Hautzellen, Muskelzellen oder Blutgefäße handeln. Werden zum Beispiel Hautzellen durch eine solche Oberflächenstruktur eines transkutanen Implantats in einer einheitlichen Richtung orientiert, so führt die so durch die Oberflächenstruktur geförderte entstandene Zellanordnung zu einer beschleunigten Wundheilung und zu einer verbesserten Verbindung zwischen den umliegenden Gewebezellen und der Implantatwand, insbesondere dem strukturierten Bereich der Oberfläche des Implantats. Eine solche verbesserte Verbindung zwischen umliegenden Gewebezellen und Implantat führt auch zu einem verbesserten Schutz vor Bakterien und ungewünschten Stoffen, so dass das Infektions- und Entzündungsrisiko beim Einsetzen des Implantats verringert werden kann. Durch eine geeignete Oberflächenstruktur kann eine dichtere Besiedelung der Implantatoberfläche durch das umliegende Gewebe und somit eine verbesserte Haftung der Zellen an der Implantatwand erreicht werden.

Das Implantat ist bevorzugt zumindest zum Teil aus einem beständigen nichtresorbierbaren Material, wie zum Beispiel Metall oder Kunststoff oder Kombinationen daraus hergestellt, so dass das Implantat nach dem Einsetzen nicht abgebaut wird.

Vorteilhaft weist das Implantat eine geeignete Oberflächenstruktur im Bereich der Hautdurchdringung auf, wobei die Oberflächenstruktur besonders bevorzugt in etwa parallel zu der Hautoberfläche im eingesetzten Zustand des Implantats verläuft.

Das Vorsehen einer Oberflächenstruktur, welche ein Anwachsen von Zellstrukturen, insbesondere Blutgefäßen parallel zum vorhandenen Gewebe, also zum Beispiel parallel zur Hautoberfläche fördert, führt auch dazu, dass sich die Blutgefäße hauptsächlich parallel zur Hautoberfläche um das Implantat herum und vom Implantat weg anlagern und im Wesentlichen nicht entlang der Implantatwand in die Tiefe reichen. Eine solche Anordnung der Blutgefäße um das Implantat herum führt zur besseren Durchblutung des umliegenden Gewebes und ist weiterhin vorteilhaft, da sich eventuell an der Hautoberfläche auftretende Infektionen nur schwer in tiefere Regionen entlang der Implantatwand fortpflanzen können. Infektionen können daher effizienter durch körpereigene Abwehrkräfte bekämpft werden und werden weiterhin durch den Patienten früher erkannt.

Vorteilhaft ist die Struktur auf der Oberfläche des Implantats 1 bis 10 mm breit, besonders bevorzugt 4 bis 5 mm breit, so dass entlang dieser bevorzugten Breite der Oberflächenstruktur umliegendes Gewebe bei dem eingesetzten Implantat in einer Tiefe von 1 bis 10 mm, bevorzugt 4 bis 5 mm orientiert und gerichtet an das Implantat anwachsen kann.

Vorteilhaft ist die Oberflächenstruktur um mindestens einen Teilbereich des Implantats umlaufend angeordnet, zum Beispiel bei einem kreis- oder ovalförmigen Querschnitt des Implantats im Bereich des Durchtritts durch die Hautoberfläche als um das Implantat umlaufende Rillenstruktur ausgestaltet, welche bevorzugt eine einheitliche Orientierung aufweist, d. h. die einzelnen Rillen sind bevorzugt in etwa parallel zueinander.

Besonders bevorzugt ist die Oberflächenstruktur als Rillenstruktur, zum Beispiel als eine umlaufende spiral- oder schraubenförmige Rille ausgestaltet. Jedoch kann die Oberflächenstruktur auch nur auf bestimmten Bereichen des Implantats vorgesehen sein, wobei andere Oberflächenbereiche des Implantats keine Oberflächenstruktur aufweisen, so dass zum Beispiel nur einzelne Rillen oder Rillenstrukturabschnitte auf der Implantatoberfläche vorgesehen sind, welche zum Beispiel durch eine glatte Implantatoberfläche durchbrochen werden, wenn zum Beispiel eine gewünschte Orientierung des Zellwachstums nur in einem Bereich der Implantatoberfläche gewünscht wird.

Die Tiefe der Rillen ist bevorzugt im Bereich des 0,1 bis 10-fachen, besonders bevorzugt im Bereich des 0,3 bis 5-fachen der durchschnittlichen Breite des Zelltyps, welcher an die Rille anwachsen soll, wobei unter der durchschnittlichen Breite des Zellentyps die Breite verstanden wird, wenn die Zelle auf eine flache Oberfläche gelegt wird.
Vorteilhaft liegt die Tiefe einer oder aller Rillen im Bereich von 1 bis 10 µm, bevorzugt im Bereich von 3 bis 4 µm.

Vorteilhaft liegt die Breite einer Rille im Bereich von 1 bis 10 µm, besonders bevorzugt im Bereich von 4 bis 5 µm.

Der Rillenabstand, d. h. der Abstand von Rillenmitte zu Rillenmitte liegt, vorteilhaft im Bereich von 2 bis 20 µm und ist bevorzugt 10 µm.

Vorteilhaft ist das Verhältnis von Breite der Rille zu der Tiefe der Rille im Bereich von 0,5 bis 2, vorzugsweise im Wesentlichen 1:1.

Bevorzugt sind auf der Implantatoberfläche mindestens zwei, bevorzugt drei bis zehn oder mehr Bereiche mit einer unterschiedlichen Oberflächenstruktur vorgesehen, so dass zum Beispiel eine erste Oberflächenstruktur im Bereich nahe der Hautoberfläche beim eingesetzten Implantat vorgesehen ist, um das Anwachsen von Hautzellen zu ermöglichen und eine oder mehrere andere Oberflächenstrukturen unterhalb dieser ersten Oberflächenstruktur vorgesehen sind, welche das Anwachsen von unter der Hautoberfläche liegenden Zellstrukturen, wie zum Beispiel Blutgefäßen oder ähnlichem fördern.

Vorteilhaft ist am Implantat eine untere Haltestruktur vorgesehen, welche in das Gewebe eingesetzt wird und zum Beispiel ein um die Unterseite des Implantats umlaufender und vom Implantat vorstehender Ring ist, um den Halt des eingesetzten Implantats zu fördern. Dabei können vorteilhaft an einer oder mehreren Stellen des umlaufenden Ringes Durchgänge oder Löcher vorgesehen sein, durch welche Gewebe hindurchwachsen kann, um so einen besseren Sitz des Implantats zu fördern. Vorteilhaft kann im Ansatzbereich des umlaufenden Ringes eine Vertiefung, zum Beispiel eine Rille vorgesehen sein, in welche umliegendes Gewebe einwachsen kann, so dass der Sitz des Implantats weiter verbessert wird.

Bevorzugt ist das Implantat ein transkutanes Implantat, wie zum Beispiel ein Portkörper und weist im oberen Bereich, also zum Beispiel in dem Bereich, welcher aus dem Gewebe beim eingesetzten Zustand des Implantats herausragt, eine Verbindungsstruktur, wie zum Beispiel ein Gewinde oder geeignete Halteelemente, wie zum Beispiel Vertiefungen mit Vorsprüngen auf, an welchen ein Verbindungselement angebracht werden kann, um von einer externen Quelle eine Substanz durch den Portkörper hindurch von außen in ein Gewebe einführen zu können, wobei der Portkörper zum Beispiel eine Öffnung an seiner Unterseite haben kann, durch die eine von der Oberseite eingebrachte Substanz in das Gewebe eintritt.

Gemäß einem weiteren Aspekt der vorliegenden Erfindung wird ein Verfahren zur Herstellung des oben beschriebenen Implantats vorgeschlagen, wobei eine Oberflächenstruktur, welche das Einwachsverhalten des Implantats verbessert, auf zumindest einem Teilbereich der Oberfläche des Implantats erzeugt wird.

Dies kann zum Beispiel durch mechanisches Erzeugen einer Rillenstruktur auf der Oberfläche erfolgen, wobei zum Beispiel eine Spiralrille direkt auf der Oberfläche des Implantats so erzeugt wird, dass die Rille spiralenförmig um zum Beispiel eine zylindrische Implantatwand herum führt. Diese Spiralrille kann zum Beispiel mit einem speziell geschliffenen Formstahl in einem Arbeitsgang im Drehverfahren erzeugt werden. Es ist vorteilhaft das Implantat nach dem Erzeugen der Rille entlang der erzeugten Rille oder Rillen zu polieren, so dass die Oberfläche im Wesentlichen glatt wird. Die Rillen werden hierbei abgerundet, bleiben aber trotzdem erhalten. Anschließend kann das Implantat vorteilhaft anodisiert oder eloxiert werden, wobei eine Oxidschicht ausgebildet wird, welche sich auch über den strukturierten Bereich des Implantats erstreckt.

Ebenso ist es möglich ein Ätzverfahren zu verwenden, um eine gewünschte Oberflächenstruktur zum Beispiel mittels einer aufgebrachten Ätzmaske zu erzeugen. Dabei werden Oberflächenbereiche, welche nicht geätzt werden sollen, mit einer säureresistenten Schicht geschützt. Mit einem Ätzverfahren kann eine Vielzahl von unterschiedlichen Oberflächenstrukturen in einem Arbeitsgang erzeugt werden.

Die Erfindung wird nachfolgend anhand eines bevorzugten Ausführungsbeispiels beschrieben werden. Dabei zeigt:
- Figur 1: ein Implantat mit einem vergrößerten Teilbereich der Oberfläche;
- Figur 2: einen im Gewebe eingesetzten Portkörper mit einer erfindungsgemäßen Oberflächenstruktur; und
- Figur 3: eine Vergrößerung eines Teilbereichs des in Figur 2 gezeigten Portkörpers.

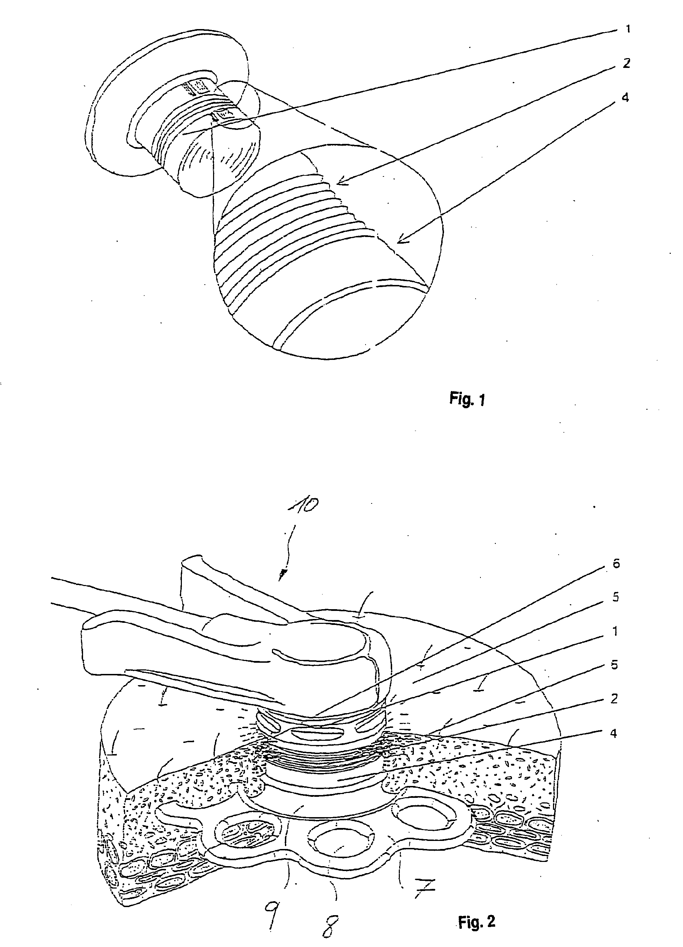

Figur 1 zeigt ein Implantat 1, welches an einem Bereich der Implantatwand 4 einen strukturierten Oberflächenbereich 2 in Form von umlaufenden Rillen aufweist. Wird das Implantat 1 so im Gewebe eingesetzt, dass die Rillen 2 im Bereich der Hautoberfläche und im Wesentlichen parallel zur Hautoberfläche liegen, so können die umlaufenden Rillen das Anwachsen der Hautzellen in einer Richtung entlang der Rillen fördern und somit zu einem besseren Einwachsen und sichereren Halt des Implantats beitragen.

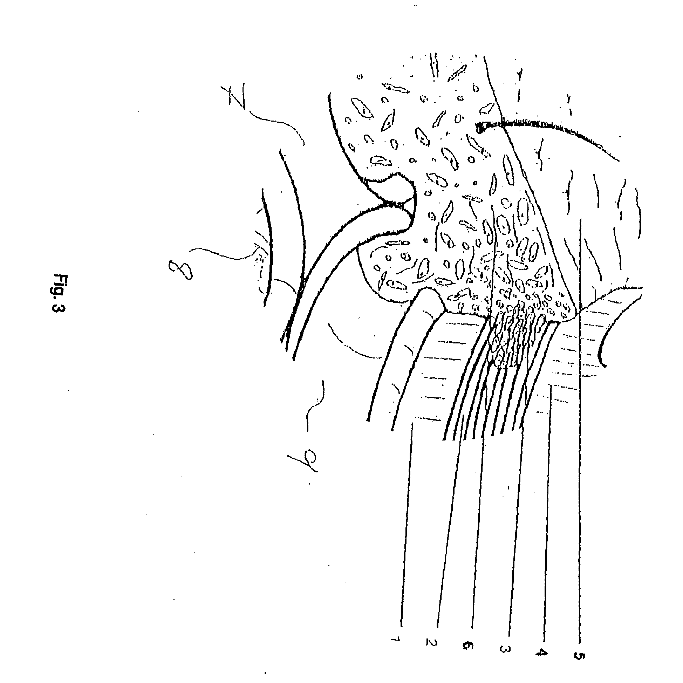

Figur 2 zeigt einen in ein Gewebe eingesetzten Portkörper 1 mit einer umlaufenden Oberflächenstruktur 2 in Form einer umlaufenden Spiralrille. Wie insbesondere aus Figur 3 ersichtlich lagern sich Gewebezellen 3, wie zum Beispiel Haut- und Muskelzellen sowie auch Blutgefäße 6 entlang der gerichteten Rillenstruktur 2 an, wodurch parallel zur Hautoberfläche eine dichte Besiedelung der Portkörperoberfläche 4 durch das Gewebe 3 erzielt werden kann. Eine solche dichtere Besiedelung durch Gewebezellen führt zu einer verbesserten Verbindung, d. h. zu einem besseren Einwachsen des umliegenden Gewebes 3 in den Portkörper 1, wodurch ein sicherer und festerer Halt des Portkörpers 1 geschaffen werden kann. Die so durch die umlaufende Rillenstruktur 2 ausgerichtete Zellstruktur regt auch die Blutgefäße 6 dazu an, sich parallel zum vorhandenen Gewebe 3 zu orientieren, so dass die Blutgefäße 6 überwiegend hauptsächlich parallel zur Hautoberfläche 5 um den Portkörper 1 herum verlaufen. Hierdurch wird die Durchblutung in dem den Portkörper 1 umgebenden Gewebebereich gefördert und das Ausbreiten einer Infektion in die Tiefe des Gewebes weitgehend verhindert.

Unterhalb der strukturierten Oberfläche 2 des Portkörpers 1 ist eine im Wesentlichen an der Oberfläche glatte Implantatwand 4 ausgebildet, an welcher umliegendes Gewebe einfach anwächst oder anliegt, wie bei den Implantaten aus dem Stand der Technik bekannt. Ein um die Unterseite des Portkörpers 1 umlaufender und vorstehender Ring 7 sorgt für einen besseren Halt des eingesetzten Implantats 1 im Gewebe 3. Der Ring 7 wird durch mehrere Löcher 8 unterbrochen, durch welche Gewebe 3 hindurchwachsen kann, um den Halt des Portkörpers 1 im Gewebe 3 weiter zu verbessern. Am Ansatzbereich des Ringes 7 am Portkörper 1 ist eine Rille vorgesehen, in welche Gewebe 3 einwachsen kann.

Der aus dem Gewebe an der Hautoberfläche 5 vorstehende Bereich des Portkörpers 1 weist eine Verbindungsstruktur (nicht gezeigt) auf, an welcher ein Verbindungselement 10 angebracht werden kann, um eine Substanz von einem externen Vorratsbehälter durch den Implantatkörper 1 hindurch in das Gewebe 3 einzuführen. Dabei kann der Portkörper 1 an seiner Unterseite eine Öffnung (nicht gezeigt) aufweisen, durch welche die Substanz von dem Portkörper 1 in umliegendes Gewebe abgegeben wird.

## Patentansprüche

1. Transkutanes Implantat mit einer Oberflächenstruktur (2) auf mindestens einer Teiloberfläche des Implantats, die das Einwachsverhalten des Implantats (1) verbessert, wobei
die Oberflächenstruktur (2) in einem Bereich des Implantats (1) vorgesehen ist, welcher im eingesetzten Zustand des Implantats (1) im Wesentlichen im Bereich der Hautdurchdringung liegt, und wobei die Oberflächenstruktur mindestens eine Rille aufweist
**dadurch gekennzeichnet, dass**
die Tiefe der mindestens einen Rille in dem Bereich von 1 bis 10 µm liegt.

2. Transkutanes Implantat nach Anspruch 1, wobei das Implantat (1) aus nichtresorbierbarem Material, insbesondere Metall und/oder Kunststoff besteht.

3. Transkutanes Implantat nach einem der vorhergehenden Ansprüche, wobei die Oberflächenstruktur (2) eine Breite von 1 bis 10 mm, bevorzugt 4 bis 5 mm aufweist.

4. Transkutanes Implantat nach einem der vorhergehenden Ansprüche, wobei an mindestens einem Bereich der Oberfläche des Implantats (1) eine umlaufende Oberflächenstruktur vorgesehen ist.

5. Transkutanes Implantat nach einem der vorhergehenden Ansprüche, wobei die Oberflächenstruktur (2) mehrere Rillen sind.

6. Transkutanes Implantat nach Anspruch 5, wobei der Abstand der Rillen voneinander in dem Bereich von 2 bis 20 µm liegt und bevorzugt 10 µm ist.

7. Transkutanes Implantat nach einem der vorhergehenden Ansprüche, wobei die Tiefe der mindestens einen Rille im Bereich des 0,1 bis 10-fachen, bevorzugt 0,3 bis 5-fachen der durchschnittlichen Breite des Zelltyps liegt, welcher daran anwachsen soll.

8. Transkutanes Implantat nach einem der vorhergehenden Ansprüche, wobei die Tiefe der mindestens einen Rille im Bereich von 3 bis 4 µm liegt.

9. Transkutanes Implantat nach einem der vorhergehenden Ansprüche, wobei die Breite der mindestens einen Rille in dem Bereich von 1 bis 10µm, bevorzugt 4 bis 5 µm liegt.

10. Transkutanes Implantat nach einem der vorhergehenden Ansprüche, wobei das Verhältnis der Rillenbreite zur Rillentiefe im Bereich von 0,5 bis 2 liegt.

11. Transkutanes Implantat nach einem der vorhergehenden Ansprüche, wobei mindestens zwei unterschiedliche Bereiche auf der Implantatoberfläche mit einer Oberflächenstruktur (2) vorgesehen sind.

12. Transkutanes Implantat nach einem der vorhergehenden Ansprüche, wobei eine Haltestruktur, insbesondere eine Ringstruktur (7) mit bevorzugt mindestens einem Durchgang (8) vorgesehen ist.

13. Transkutanes Implantat nach einem der vorhergehenden Ansprüche, wobei das Implantat ein Portkörper ist.

14. Verfahren zur Herstellung eines transkutanen Implantats nach einem der vorhergehenden Ansprüche, wobei auf mindestens einem Oberflächenbereich des Implantats (1) eine Oberflächenstruktur (2) erzeugt wird, welche das Einwachsverhalten des Implantats an umliegendem Gewebe verbessert, wobei die Oberflächenstruktur (2) in einem Bereich des Implantats (1) vorgesehen wird, welcher im eingesetzten Zustand des Implantats (1) im Wesentlichen im Bereich der Hautdurchdringung liegt, und wobei die Oberflächenstruktur mindestens eine Rille aufweist und die Tiefe der mindestens einen Rille im Bereich von 1 bis 10 µm liegt.

15. Verfahren nach Anspruch 14, wobei die Oberflächenstruktur (2) durch Drehen einer Rille geschaffen wird.

16. Verfahren nach Anspruch 14, wobei die Oberflächenstruktur (2) durch Ätzen geschaffen wird.

## Claims

1. Transcutaneous implant with a surface structure (2) on at least one partial region of the implant, which improves the growing-in behaviour of the implant (1), wherein the surface structure (2) is provided in a region of the implant (1), which in the inserted state of the implant (1) lies essentially in the area where it penetrates the skin, and wherein the surface structure has at least one groove, **characterised in that** the depth of the at least one groove lies in the range from 1 to 10 µm.

2. Transcutaneous implant according to Claim 1, wherein the implant (1) is made of non-resorbable material, in particular metal and/or plastic.

3. Transcutaneous implant according to one of the preceding claims, wherein the surface structure (2) has a width of 1 to 10 mm, preferably 4 to 5 mm.

4. Transcutaneous implant according to one of the preceding claims, wherein a circumferential surface structure is provided on at least one region of the surface of the implant (1).

5. Transcutaneous implant according to one of the preceding claims, wherein the surface structure (2) consists of several grooves.

6. Transcutaneous implant according to Claim 5, wherein the spacing of the grooves from one another lies in the range from 2 to 20 µm and is preferably 10 µm.

7. Transcutaneous implant according to one of the preceding claims, wherein the depth of the at least one groove lies in the range of 0.1 to 10 times, preferably 0.3 to 5 times the average width of the cell type, which is to grow on it.

8. Transcutaneous implant according to one of the preceding claims, wherein the depth of the at least one groove lies in the range from 3 to 4 µm.

9. Transcutaneous implant according to one of the preceding claims, wherein the width of the at least one groove lies in the range from 1 to 10 µm, preferably 4 to 5 µm.

10. Transcutaneous implant according to one of the preceding claims, wherein the ratio of the groove width to the groove depth lies in the range from 0.5 to 2.

11. Transcutaneous implant according to one of the preceding claims, wherein at least two different areas on the implant surface are provided with a surface structure (2).

12. Transcutaneous implant according to one of the preceding claims, wherein a retaining structure, in particular a ring structure (7) with preferably at least one aperture (8) is provided.

13. Transcutaneous implant according to one of the preceding claims, wherein the implant is a port body.

14. Method for producing a transcutaneous implant according to one of the preceding claims, wherein a surface structure (2), which improves the growing-in behaviour of the implant to surrounding tissue, is produced on at least one surface region of the implant (1), wherein the surface structure (2) is provided in a region of the implant (1), which in the inserted state of the implant (1) lies essentially in the area where it penetrates the skin, and wherein the surface structure has at least one groove and the depth of the at least one groove lies in the range from 1 to 10 µm.

15. Method according to Claim 14, wherein the surface structure (2) is produced by turning a groove.

16. Method according to Claim 14, wherein the surface structure (2) is produced by etching.

## Revendications

1. Implant transcutané avec une structure superficielle (2) sur au moins une surface supérieure partielle de l'implant, qui améliore le comportement de croissance de l'implant (1), dans lequel
la structure superficielle (2) est prévue dans une zone de l'implant (1) qui, dans l'état mis en place de l'implant (1), se situe sensiblement dans une zone de pénétration de la peau, et dans lequel la structure superficielle présente au moins une gorge,
**caractérisé en ce que**
la profondeur de ladite moins une gorge se situe dans la plage de 1 à 10 µm.

2. Implant transcutané selon la revendication 1, dans lequel l'implant (1) consiste en un matériau non résorbable, en particulier un métal et/ou une matière synthétique.

3. Implant transcutané selon l'une des revendications précédentes, dans lequel la structure superficielle (2) présente une largeur de 1 à 10 mm, de préférence de 4 à 5 mm.

4. Implant transcutané selon l'une des revendications précédentes, dans lequel, sur au moins une zone de la surface supérieure de l'implant, est prévue une structure superficielle périphérique.

5. Implant transcutané selon l'une des revendications précédentes, dans lequel la structure superficielle (2) comporte plusieurs gorges.

6. Implant transcutané selon la revendication 5, dans lequel la distance des gorges entre elles se situe dans la plage de 2 à 20 µm, et de préférence de 10 µm.

7. Implant transcutané selon l'une des revendications précédentes, dans lequel la profondeur de ladite au moins une gorge se situe dans la plage de 0,1 à 10 fois, de préférence 0,3 à 5 fois la largeur moyenne du type de cellule qui doit y pousser.

8. Implant transcutané selon l'une des revendications précédentes, dans lequel la profondeur de ladite au moins une gorge se situe dans la plage de 3 à 4 µm.

9. Implant transcutané selon l'une des revendications précédentes, dans lequel la largeur de ladite au moins une gorge se situe dans la plage de 1 à 10 µm, de préférence de 4 à 5 µm.

10. Implant transcutané selon l'une des revendications précédentes, dans lequel le rapport entre la largeur de gorge et la profondeur de gorge se situe dans la plage de 0,5 à 2.

11. Implant transcutané selon l'une des revendications précédentes, dans lequel au moins deux zones différentes sur la surface supérieure d'implant sont munies d'une structure superficielle (2).

12. Implant transcutané selon l'une des revendications précédentes, dans lequel une structure de retenue, en particulier une structure circulaire (7), est de préférence munie d'au moins un passage (8).

13. Implant transcutané selon l'une des revendications précédentes, dans lequel l'implant est un corps poreux.

14. Procédé pour la préparation d'un implant transcutané selon l'une des revendications précédentes, dans lequel, sur au moins une zone superficielle de l'implant (1), est produite une structure superficielle (2) qui améliore le comportement de croissance de l'implant sur un tissu avoisinant, dans lequel la structure superficielle (2) est prévue dans une zone de l'implant (1) qui, à l'état mis en place de l'implant (1), se situe sensiblement dans la zone de pénétration de la peau et dans lequel la structure superficielle présente au moins une gorge, et la profondeur de ladite au moins une gorge se situe dans la plage de 1 à 10 µm.

15. Procédé selon la revendication 14, dans lequel la structure superficielle (2) est produite par rotation d'une gorge.

16. Procédé selon la revendication 14, dans lequel la structure superficielle (2) est produite par attaque corrosive.
